# EUROPÄISCHE PATENTSCHRIFT

(11) **EP 1 212 008 B1**
(45) Veröffentlichungstag und Bekanntmachung des Hinweises auf die Patenterteilung: **16.06.2004**
(21) Anmeldenummer: 00967641.2
(22) Anmeldetag: 01.09.2000
(51) Int. Cl.: A61F 2/08, A61B 17/17

(54) **INSTRUMENTARIUM ZUM IMPLANTIEREN EINES SEHNENERSATZES**
INSTRUMENTARIUM FOR IMPLANTING A TENDON TRANSPLANT
INSTRUMENTATION POUR L'IMPLANTATION D'UN TENDON DE REMPLACEMENT

(30) Priorität: 01.09.1999 DE 19941574
(43) Veröffentlichungstag der Anmeldung: 12.06.2002
(73) Patentinhaber: Karl Storz GmbH & Co., 78532 Tuttlingen (DE)
(72) Erfinder: STROBEL, Michael, 94315 Straubing (DE); SAUER, Michael, 78532 Tuttlingen (DE)
(74) Vertreter: Weller, Wolfgang, Dr.
(86) Internationale Anmeldenummer: PCT/EP2000/008567
(87) Internationale Veröffentlichungsnummer: WO 2001/015634

(56) Entgegenhaltungen:
- EP-A- 0 495 487
- EP-A- 0 865 774
- WO-A-92/02196
- WO-A-92/16167
- DE-U- 29 607 352
- GB-A- 2 288 739
- US-A- 5 306 301

## Beschreibung

Die Erfindung betrifft ein Instrumentarium zum Implantieren eines Sehnenersatzes, insbesondere eines Kreuzbandersatzes in einem Kniegelenk, mit einem Button, der an eine äußere Mündung eines Knochenkanales anlegbar ist, in dem der Sehnenersatz angeordnet ist, wobei der Button Öffnungen aufweist, durch die Fixationsfäden des Sehnenersatzes hindurchziehbar sind, wobei der Button einen mittigen Vorsprung aufweist, der bei angelegtem Button in die Mündung des Knochenkanales hineinragt.

Ein derartiges Instrumentarium und die entsprechende Operationstechnik, bei der dieses Instrumentarium eingesetzt wird, ist aus dem Lehrbuch "Michael Strobel; Arthroskopische Chirurgie", Seiten 398 bis 444 "Operationstechnik", Springer-Verlag Berlin Heidelberg 1998, bekannt. Ein solches Instrumentarium ist auch in der deutschen Patentanmeldung DE-A-198 51 152, angemeldet am 6. November 1998 und veröffentlicht am 11.05.2000 der Anmelderin beschrieben.

Zum Implantieren eines Kreuzbandersatzes in einem Kniegelenk wird ein Bohrkanal bei einem Flexionswinkel des Kniegelenkes von etwa 60 bis 90° bewerkstelligt, der sowohl durch das distale Ende des Femurs als auch durch das proximale Ende der Tibia hindurch reicht. In diesen Bohrkanal wird der Sehnenersatz eingesetzt, wobei die Sehne eine natürliche Sehne des Patienten ist, bspw. die Semitendinosus-Sehne. An die Enden der Sehnen sind Fixationsfäden angenäht, die beidseits aus dem Bohrkanal hinausreichen und zur Fixierung der Sehne dienen. Die an der Außenseite des Femurs hinausreichenden Fäden werden üblicherweise mittels einer Platte, die an der Mündung des Bohrkanales anliegt, fixiert. Die am gegenüberliegenden Ende des Bohrkanales, also an der Außenseite der Tibia austretenden Fixationsfäden, werden über einen sogenannten tibialen Button fixiert.

Dieser Button hat in etwa die Form und Größe eines Hemdknopfes, d.h. er hat einen etwa scheibenförmigen oder tellerrandförmigen runden Grundkörper, von dem von einer Seite eine mittige Wölbung vorspringt und der an der gegenüberliegenden Seite entsprechend eingesenkt ist. In diesem Bereich befinden sich Öffnungen, durch die die Fixationsfäden hindurchgezogen werden können. Ein solcher Button wird so an den Bohrkanal angesetzt, daß dessen Vorsprung oder Auswölbung in den Bohrkanal teilweise hineinreicht. Die Fixationsfäden werden durch die Öffnungen bzw. Löcher hindurchgezogen und verknotet, dieser Vorgang ist in der Bildfolge in Strobel, a.a.O., Seite 417, dargestellt. Die Kontur eines solchen Buttons ist, von der Seite her gesehen, derart, daß vom äußeren Rand her gesehen der Vorsprung in einer sanften Krümmung zu einem abgerundeten Buckel ansteigt und auf der diametral gegenüberliegenden Seite entsprechend symmetrisch wieder in einer sanften S-Linie abfällt.

Nachteilig an einem derartigen Button ist, daß er im Bereich seines mittigen Vorsprunges nur etwa längs der Umfangskante der Mündung des Bohrkanales anliegt, dementsprechend bei Belastung verrutschen, insbesondere auch verkippen kann.

Sind die durch die Löcher des Buttons hindurchgezogenen Fixationsfäden an der Außenseite verknotet, kann man, um der Sehne etwas mehr Spannung zu verleihen, den so fixierten Button etwas drehen. Durch den zuvor beschriebenen Sitz des Buttons lediglich über die äußere Umfangslinie der Mündungsöffnung des Bohrkanales ist nicht sichergestellt, daß dabei ein ausreichender Reibschluß gewährleistet ist, der ein Rückdrehen und somit wieder Entspannen der Sehne ausschließt.

Aus der EP-A-0 865 774 ist ein Verankerungselement zur Verbindung mit Fixationsfäden eines Sehnenersatzes bekannt, das als länglicher Körper ausgebildet ist, der sich diametral über die Mündungsöffnung des Bohrkanales hinweg erstreckt und diesen beidseits überragt. Von den die Mündungsöffnung beidseits überragenden Abschnitten des Körpers stehen nagelartige Elemente in Richtung des Knochens vor und dienen dazu, in diesen eingetrieben zu werden. Dieses Verankerungselement wird also quer über die Mündungsöffnung genagelt. Im Bereich der Mündungsöffnung weist der längliche Körper eine V-förmige Einsenkung auf, die in den Bohrkanal hineinreicht. In der Rille des "Vs" werden die Fixationsenden eingelegt und verknotet. Nachteilig daran ist, daß ein Drehen des Verankerungselementes, nachdem die Fixationsfäden mit diesem verknotet sind, um die Sehne zu spannen, nicht möglich ist, da dies über die in den Knochen eingetriebenen nagelartigen Stifte drehfest fixiert ist. Das Verankerungselement ist darüber hinaus kompliziert ausgeformt und entsprechend aufwendig herzustellen.

Ein weiteres Verankerumgselement ist durch die Druckschrift DE-U-296 07 352 bekannt geworden.

Aufgabe der vorliegenden Erfindung ist daher, ein Instrumentarium zum Implantieren eines Sehnenersatzes dahingehend zu verbessern, daß der Sehnenersatz einfach, dennoch zutreffend und sicher sitzend implantiert werden kann.

Erfindungsgemäß wird die Aufgabe dadurch gelöst, daß der Vorsprung des Buttons als zapfenartiger Hals ausgebildet ist, der in eine diesem angepaßte Einsenkung an der Mündung des Knochenkanals einsetzbar ist.

Diese Maßnahme hat den erheblichen Vorteil, daß der als zapfenartiger Hals ausgebildete Vorsprung exakt passend in der Mündungsöffnung des Knochenkanales sitzt. Dazu wird die Mündung des Knochenkanales mit einer entsprechend dem zapfenartigen Hals angepaßten Einsenkung versehen. Durch diesen Paßsitz liegt der zapfenartige Hals relativ großflächig an der Innenseite des Knochenkanales im Bereich der Mündung an, so daß ein exakter Sitz gewährleistet ist, der ein Verrutschen oder Kippen des Buttons sicher ausschließt. Ein entsprechend reibschlüssiger Sitz verhindert auch ein ungewolltes Verdrehen des Buttons. Dieser sichere und exakte Sitz gewährleistet nach Verknoten mit den Fixationsfäden einen auf Dauer unverrückbaren Sitz, so daß auch bei den extremen Bewegungen und Belastungen, der die Fixierstelle bspw. beim Betätigen eines Kniegelenkes ausgesetzt ist, auf Dauer erhalten bleibt.

In einer weiteren Ausgestaltung der Erfindung ist der zapfenartige Hals als zylindrischer Zapfen ausgebildet.

Diese Maßnahme hat den Vorteil, daß diese Zylindergeometrie einfach herstellbar ist, dementsprechend auch einfach die daran angepaßte Einsenkung in der Mündung herstellbar ist und eine einfache Handhabung durch den Operateur möglich ist. Die aus dem Knochenkanal herausragenden Fixationsfäden brauchen lediglich durch die Öffnungen des Buttons hindurchgefädelt zu werden, dieser wird dann anschließend längs der gestrafften Fäden in Richtung Mündung des Knochenkanales verschoben und kann dann exakt ausgerichtet in die Einsenkung eingeschoben werden. Die Zylindergeometrie erlaubt ein Drehen des bereits in den Knochenkanal eingesetzten Buttons, sei es um die Ausrichtung zu korrigieren, oder später, nach Verknoten mit den Fixationsfäden, um durch ein Drehen ein nachträgliches Spannen des Sehnenersatzes zu erzielen.

In einer weiteren Ausgestaltung der Erfindung weist der Button einen plattenförmigen Körper auf, von dem der zapfenartige Hals vorspringt.

Diese Maßnahme hat den Vorteil, daß der Button nicht nur über den zylindrischen Zapfen exakt in der Mündung des Knochenkanales sitzt, sondern über den plattenförmigen Körper eine weitere relativ große Anlagefläche geschaffen ist, über die der Button an der Außenseite des Knochens anliegt.

Durch die Spannung der über die Fixationsfäden mit dem Button verknoteten Sehne ist ein ausreichender Reibschluß gegeben, damit der Button drehsicher sitzt. Dennoch ist, insbesondere in Zusammenhang mit der Zylindergeometrie, die Möglichkeit gegeben, unter Einsatz eines später zu beschreibenden entsprechenden Werkzeuges den Button unter Überwindung dieses Reibschlusses zu drehen, sei es um die Spannung der Sehne zu erhöhen oder zu lockern.

In einer weiteren Ausgestaltung ist vorgesehen, daß der plattenförmige Körper unrund ist.

Die Ausbildung einer unrunden Geometrie eröffnet die Möglichkeit, an dem plattenförmigen Körper ein Werkzeug im Formschluß und nicht nur im Reibschluß anzusetzen, wie das bei der Kreisgeometrie des Buttonkörpers beim eingangs genannten Stand der Technik der Fall ist. Soll ein kreisrunder scheibenförmiger Körper gedreht werden, müßte ein Werkzeug mit einer solchen Faßkraft angesetzt werden, daß Beschädigungen des doch relativ kleinen Bauteiles nicht ausgeschlossen werden können. Dazu ist zu beachten, daß die Zugkraft einer Sehne, die auf einen solchen Button einwirkt, erheblich ist.

In einer weiteren Ausgestaltung der Erfindung weist der plattenförmige Körper Kanten auf, an die ein Werkzeug zum Drehen des Buttons ansetzbar ist.

Diese Maßnahme hat den Vorteil, daß diese Kanten dem Operateur einfach ersichtliche Orientierungsmerkmale darstellen, um ein solches Werkzeug anzusetzen, so daß die Handhabung wesentlich erleichtert ist.

In einer weiteren Ausgestaltung der Erfindung weist der plattenförmige Körper zwei gegenüberliegende Kanten auf.

Diese Gestaltungsmerkmale erleichtern noch wesentlicher die Handhabung des Buttons, insbesondere beim Drehen und erlauben eine gleichmäßige symmetrische Verteilung der Kräfte, die beim Drehen auf den Button einwirken.

In einer weiteren Ausgestaltung der Erfindung weist der Button auf der Seite, die dem zapfenartigen Hals gegenüberliegt, im Bereich der Öffnungen eine Mulde auf.

Diese Maßnahme hat den Vorteil, daß in diese Mulde die Knoten der Fixationsfäden eingelegt werden können bzw. diese Knoten orientiert und lagefest zum Liegen kommen.

In einer weiteren Ausgestaltung der Erfindung geht die Mulde in sanften Krümmungen in die Öffnung über.

Diese Maßnahme hat den Vorteil, daß die beim Dehnen und Verkürzen der Sehne, bspw. bei der Bewegung eines Kniegelenks, verbundenen Scheuerbewegungen zwischen den Fixationsfäden und dem Button nicht dazu führen können, daß diese durch Kanten nach und nach durchgescheuert werden und die Verbindung gelöst wird.

In einer weiteren Ausgestaltung der Erfindung sind zwei Öffnungen im Button vorhanden.

Diese einfache Ausgestaltung erlaubt, auch mehrere Fäden miteinander sicher und fest zu verknoten. Dazu reicht es aus, daß ein Materialsteg zwischen den beiden Öffnungen vorhanden ist, so daß bspw. auch vier Fäden oder sechs Fäden, die durch die beiden Öffnungen hindurchgezogen sind, entsprechend festsitzend miteinander verknotet werden können.

In einer weiteren Ausgestaltung der Erfindung springt der zapfenartige Hals von einer flächigen Seite des Buttons vor.

Diese Maßnahme hat den Vorteil, daß diese flächige Seite eine relativ große Anlagefläche des plattenförmigen Körpers am Button eröffnet, so daß der Button an dieser Seite, an der der zapfenartige Hals vorspringt, praktisch über seine ganze Fläche an dem Knochen anliegt, einmal mit der flächigen Unterseite an der Außenseite des Knochens und mit dem in den Knochenkanal hineinreichenden zapfenartigen Hals an der Innenseite des Knochenkanales. Damit ist nicht nur ein besonders hervorragender Sitz sondern auch eine ausreichende Anlegefläche geschaffen, um einen drehsicheren Reibschluß zu gewährleisten, d.h. drehsicher gegenüber den bei üblichen Belastungen auftretenden Kräften.

In einer weiteren Ausgestaltung der Erfindung springt der zapfenartige Hals unter einem solchen Winkel von der flächigen Seite vor, daß bei angelegtem Button diese Seite flächig an der Knochenfläche, die den Knochenkanal umgibt, anliegt und der zapfenartige Hals dabei passend in der Einsenkung sitzt.

Diese Ausgestaltung optimiert den zuvor erwähnten exakten Sitz unter Berücksichtigung des Bohrkanalwinkels zur Außenseite.

In einer weiteren Ausgestaltung ist ein Setzgerät vorgesehen, das ein distales Werkzeug aufweist, mittels dem die Einsenkung in den Knochen für den zapfenartigen Hals herstellbar ist.

Diese Maßnahme hat den Vorteil, daß durch dieses Setzgerät eine Einsenkung im Mündungsbereich des Knochenkanals bewerkstelligt werden kann, in die der zapfenartige Hals des Buttons exakt sitzend einschiebbar ist.

In einer weiteren Ausgestaltung der Erfindung weist das Werkzeug distal einen vorstehenden Zapfen auf, dessen Länge in etwa der Länge des zapfenartigen Halses des Buttons entspricht.

Diese Maßnahme hat den Vorteil, daß die Einsenkung exakt bis in die Tiefe hergestellt wird, die der Länge des zapfenartigen Halses entspricht.

Da üblicherweise der Bohrkanal unter einem nicht-rechten Winkel zu der Außenfläche des Knochens mündet, kann eine Einsenkung hergestellt werden, deren Längsachse senkrecht zur Knochenfläche steht, so daß ein Button mit einer entsprechend einfachen rechtwinkligen Geometrie zwischen Plattenkörper und vorstehenden zapfenartigen Hals eingeschoben werden kann. Dabei entsteht dann zwangsläufig am äußeren Ende der Einsenkung eine Stufe zu dem übrigen Knochenkanal. Durch diese Stufe ist eine weitere zusätzliche Anlagefläche im stirnseitigen Endbereich des zapfenartigen Halses geschaffen.

In einer weiteren Ausgestaltung der Erfindung weist der Zapfen des Werkzeuges an seinem proximalen Ende einen Anschlag auf.

Diese Maßnahme hat den Vorteil, daß die Handhabung zur Bewerkstelligung der Einsenkung für den Operateur besonders einfach ist. Er muß das Werkzeug lediglich, bis der Anschlag an den Knochenflächen zum Liegen kommt, eintreiben. Dadurch ist sichergestellt, daß keine zu tiefe Einsenkung bewerkstelligt wird, so daß dieser Vorgang insgesamt atraumatisch durchzuführen ist.

In einer weiteren Ausgestaltung der Erfindung ist der Anschlag als Ringflansch ausgebildet.

Diese Ausbildung ist konstruktiv einfach zu bewerkstelligen und führt zu einem rundherum ausgerichteten und passenden, auch unter dem richtigen Winkel stehenden Sitz der Einsenkung.

In einer weiteren Ausgestaltung der Erfindung ist die Kontur des Zapfens ein Oval.

Diese Maßnahme hat den Vorteil, daß die Mündungsöffnung der schrägen Bohrung eine ovale Kontur hat und der ovale Zapfen daher atraumatisch in den Knochen einschlagbar ist. Durch Drehen des ovalen Zapfens verdichtet sich das Knochenmaterial, und es entsteht dann eine Einsenkung mit einer kreisförmigen Konturlinie.

In einer weiteren Ausgestaltung der Erfindung weist das Werkzeug des Setzgerätes einen seitlichen Längsschlitz auf, in den die Fixationsfäden einlegbar sind.

Diese Maßnahme hat den Vorteil, daß zunächst einmal die Sehne samt den Fixationsfäden in den Bohrkanal eingelegt werden können und erst nach dieser Handhabung die Einsenkung, in die der Button eingeschoben wird, bewerkstelligt wird. In den seitlichen Schlitz können dann die Fixationsfäden eingelegt werden, so daß diese bei Einschlagen des Setzwerkzeuges nicht beschädigt, bspw. zwischen das Werkzeug und den Knochen eingeklemmt werden.

In einer weiteren Ausgestaltung des Setzgerätes sind abseits des Werkzeuges Haken zum Einhängen der Fixationsfäden vorgesehen.

Diese Maßnahme hat den weiteren handhabungstechnischen Vorteil, daß die durch den Schlitz gebündelten Fixationsfäden in die Haken eingehängt und am Setzgerät gehalten werden können, so daß diese dem Operateur nicht die Sicht oder das Operationsfeld versperren.

In einer weiteren Ausgestaltung der Erfindung ist vorgesehen, daß das Setzgerät mit einem kreuzartigen Griff versehen ist.

Diese Ausgestaltung ermöglicht zum einen, daß am kurzen hochstehenden Ende des Kreuzes ein Hammer angesetzt werden kann, um das Setzgerät einzuschlagen, über die Querstege des Kreuzes kann dann das Werkzeug von der Hand des Operateurs ergriffen und gedreht werden, um bspw. bei einem Werkzeug mit einem ovalen Querschnitt eine runde Einsenkung zu bewerkstelligen.

In einer weiteren Ausgestaltung der Erfindung ist ein Knotenhalter vorgesehen, der einen Griff und einen davon distal vorstehenden Stab aufweist, dessen distales Ende mit einer Kerbe versehen ist, um die Fixationsfäden beim Verknoten am Button zu halten.

Diese Maßnahme hat den Vorteil, daß über den Knotenhalter nicht nur die Fäden sondern auch zugleich der Button fest an dem Knochenkanal gehalten werden kann, während die Knoten geschlungen und festgezogen werden.

In einer weiteren Ausgestaltung der Erfindung ist ein Tensiometer vorgesehen, dessen distales, gegen Federkraft ausziehbares Ende mit einem Haken versehen ist, der in eine am Button ausgebildete Schlaufe aus Fixationsfäden einhängbar ist.

Bei dieser Technik wird zusätzlich eine Schlaufe am Button befestigt. In diese Schlaufe kann nun das Tensiometer eingehängt und durch Ziehen die Kraft bestimmt werden, die notwendig ist, um den Button abzuheben, was dann Rückschlüsse auf die Spannkraft der Sehne zuläßt. Je nach Ergebnis dieser Messung kann dann durch Drehen des Buttons die Spannkraft verringert oder erhöht werden. Somit kann die optimale Spannkraft eingestellt werden, die aufgrund des festen und unverrückbaren Sitzes des Buttons auf Dauer gewährleistet bleibt.

In einer weiteren Ausgestaltung der Erfindung ist ein Buttondreher vorgesehen, der an den Button ansetzbar und mittels dem der Button drehbar ist.

Diese Maßnahme hat den Vorteil, daß über dieses Spezialwerkzeug der bereits angelegte und mit den Fixationsfäden verknotete Button nachträglich noch gedreht werden kann, sei es um dessen Lage zu korrigieren, oder sei es, bspw. aufgrund des Ergebnisses einer Messung mit dem Tensiometer, die Spannung der Sehne zu korrigieren.

In einer weiteren Ausgestaltung ist vorgesehen, daß der Buttondreher Arbeitsflächen aufweist, die an die Kanten des Buttons anlegbar sind.

Diese Maßnahme hat den Vorteil, daß durch die Abstimmung von Ausgestaltungen zwischen den Kanten des Buttons und des Buttondrehers besonders einfach und sicher der Button gedreht werden kann, was insbesondere die Handhabung durch den Operateur erleichtert, und auch ohne Beeinträchtigung des Buttons ein Drehen auch bei hohen Zugkräften durch eine stark gespannte Sehne möglich ist.

Es versteht sich, daß die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert.

Es zeigen:
- Fig. 1: einen schematischen Längsschnitt eines Kniegelenkes mit einem Kreuzbandersatz;
- Fig. 2: eine perspektivische Ansicht von oben eines erfindungsgemäßen Buttons;
- Fig. 3: einen Schnitt längs der Linie III-III in Fig. 2;
- Fig. 4: eine Ansicht des Buttons von unten, also von der Seite des Knochens her, an den der Button angelegt werden soll;
- Fig. 5: eine Draufsicht auf den Button von Fig. 2;
- Fig. 6: eine teilweise geschnittene Seitenansicht eines Setzgerätes für den Button von Fig. 2;
- Fig. 7: eine stark vergrößerte Ansicht auf das stirnseitige distale Ende des Setzgerätes von Fig. 6;
- Fig. 8: eine der Darstellung von Fig. 7 entsprechende perspektivische Darstellung;
- Fig. 9: eine Seitenansicht eines Knotenhalters zur Unterstützung der Bewerkstelligung von Knoten an dem Button von Fig. 2;
- Fig. 10: ein Tensiometer zur Messung der Spannung, über die ein Button nach Verknotung mit Fixationsfäden am Knochen anliegt;
- Fig. 11: einen Buttondreher zum Drehen des in Fig. 2 dargestellten Buttons;
- Fig. 12: eine stark vergrößerte Ansicht auf das stirnseite distale Ende des Buttondrehers von Fig. 11;
- Fig. 13: eine stark vergrößerte Darstellung eines an die Mündung eines Knochenkanales angelegten Buttons, durch dessen Öffnungen vier Fixationsfäden hindurchgezogen sind; und
- Fig. 14: eine der Fig. 13 entsprechende Darstellung eines an die Mündung des Knochenkanales angelegten Buttons entsprechend dem eingangs erwähnten Stand der Technik.

Wie anhand der nachfolgenden Figurenbeschreibung ersichtlich wird, werden zur eingehenden Erläuterung des erfindungsgemäßen Instrumentariums im folgenden auch die einzelnen Schritte des der Erfindung zugrundeliegenden Verfahrens zum Implantieren eines Sehnenersatzes dargestellt.

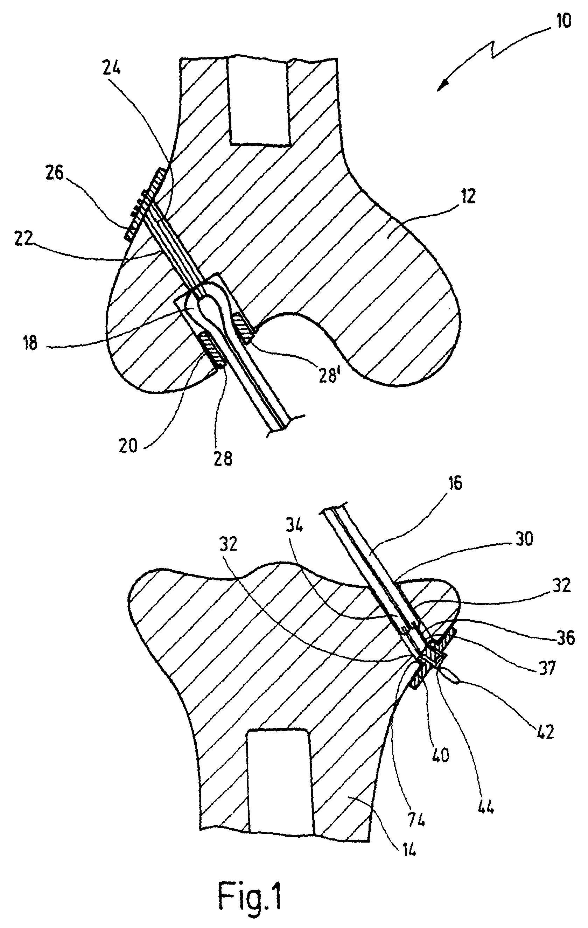

In Fig. 1 ist ein schematisch skizziertes Kniegelenk in seiner Gesamtheit mit der Bezugsziffer 10 bezeichnet.

Das Kniegelenk 10 bildet die Verbindung zwischen dem distalen Ende des Femurs 12 und dem proximalen Ende der Tibia 14.

Die beiden, die aus Gründen der Übersichtlichkeit in einem nicht-maßstabsgetreuen Abstand zueinander dargestellt sind, verbindet ein Sehnenersatz 16, im dargestellten Ausführungsbeispiel ein Ersatz des vorderen Kreuzbandes.

Die Sehne des Sehnenersatzes 16 wird aus einem Abschnitt der natürlichen Semitendinosus-Sehne des Patienten präpariert. Dazu wird ein Abschnitt dieser Sehne entnommen, zurecht geschnitten und unter Ausbildung einer Schlaufe 18 zu einem Doppelstrang gelegt.

Das Ende des Sehnenersatzes 16, das mit der Schlaufe 18 versehen ist, wird in eine Sacklochbohrung 20 im Femur 12 eingeschoben. An die Sacklochbohrung 20 schließt sich ein dünnerer Bohrkanal 22 an, der an der Außenseite des Femurs 12 mündet. Die Schlaufe 18 ist mit mehreren Fäden 24 verbunden, die durch den Bohrkanal 22 hindurch und zur Außenseite des Femurs 12 geführt werden. Dort liegt eine Platte 26 an, durch deren nicht näher bezeichnete Öffnungen die Fäden 24 hindurchgeführt und miteinander verknotet werden.

Da der Sehnenersatz 16 im Bereich der Schlaufe 18 breiter ausgebildet ist, werden noch zusätzlich Dübel 28 in die Sacklochbohrung 20 eingeschoben, um einen unverrückbaren und festen Sitz des Sehnenersatzes 16 zu gewährleisten und dessen besseres Anwachsen sicherzustellen. An dem mit der Schlaufe 18 versehenen Ende gegenüberliegenden Ende ist der Sehnenersatz 16 in eine durchgehende Bohrung 30 in der Tibia 14 eingelegt. Die äußeren Enden 34 des Sehnenersatzes 16 sind jeweils mit zwei Fixationsfäden vernäht, wobei in Fig. 1 nur die beiden Fixationsfäden 36 und 37 bezeichnet sind, die mit einem Sehnenstrang verbunden sind. Die vier Fixationsfäden reichen aus der Mündung 33 des durch die Bohrung 30 gebildeten Knochenkanales 32 heraus und sind dort mit einem Button 40 unter Ausbildung von zahlreichen Knoten 40 verbunden. Zusätzlich ist eine Schlaufe 42 angebracht, die später, nach der Anpassung, wieder entfernt wird.

Anhand der nachfolgenden Figuren soll zunächst die konstruktive Ausgestaltung des Buttons 40 sowie dessen an Ort und Stelle Bringen näher beschrieben und erläutert werden.

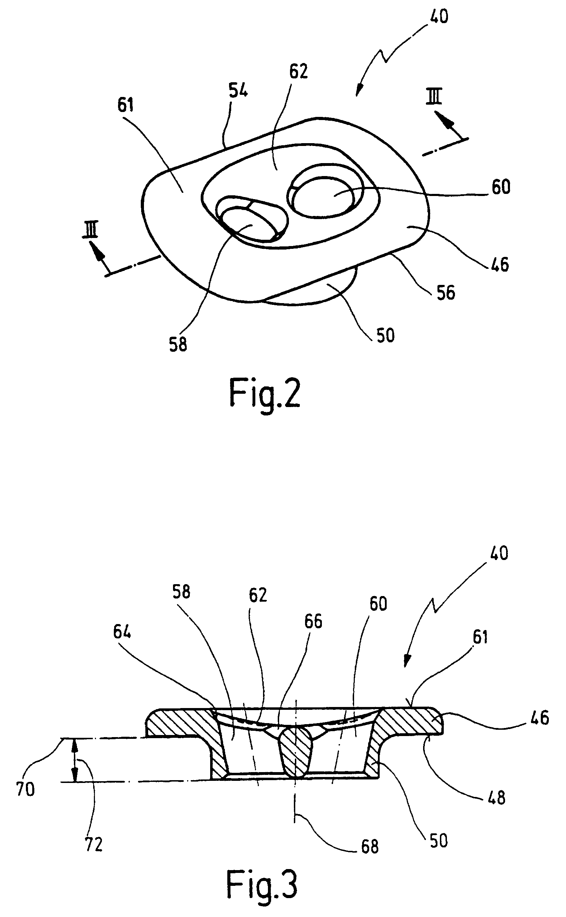

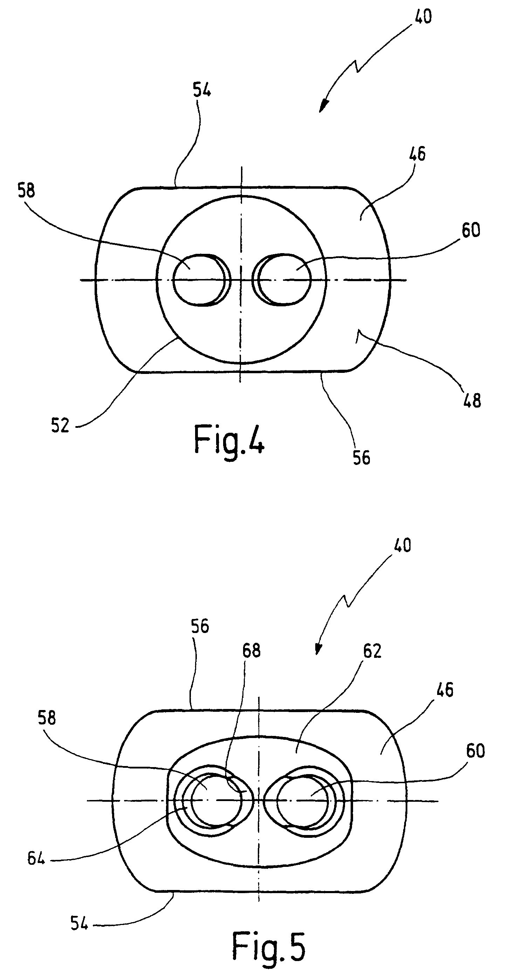

Der in Fig. 2 bis 5 näher dargestellte Button 40 weist einen etwa plattenförmigen Körper 46 auf, von dessen einer Seite 48, die der Außenseite der Tibia 14 beim Implantieren zugewandt ist, ein zapfenartiger Hals 50 vorsteht.

Der zapfenartige Hals 50 ist als zylindrischer Zapfen 52 ausgeformt.

Der Körper 46 weist zwei gegenüberliegende geradlinig und parallel zueinander verlaufende Längskanten 54 und 56 auf, deren äußere Enden über sanfte Rundungen miteinander verbunden sind, so daß in der Draufsicht etwa eine grob rechteckförmige Kontur mit gegenüberliegenden geradlinigen Kanten 54 und 56 resultiert.

Mittig sind zwei Öffnungen 58 und 60 vorgesehen, die sich durch den Körper 46 und durch den zylindrischen Zapfen 52 hindurch erstrecken, wie das insbesondere aus der Schnittdarstellung von Fig. 3 ersichtlich ist. Auf der Seite 61, die dem Zapfen 52 gegenüberliegt, ist der Körper 46 mit einer Mulde 62 versehen. Die Mulde 62 geht in sanften Krümmungen 64 bzw. 66 in die Öffnung 58 über, entsprechendes gilt für den Übergang in die Öffnung 60. Wie insbesondere aus der Schnittdarstellung von Fig. 3 ersichtlich, verläuft die Längsachse bzw. Zapfenachse 68 etwa rechtwinklig zu einer Ebene 70, die sich längs der unteren flachen ebenen Seite 48 des Körpers 46 erstreckt.

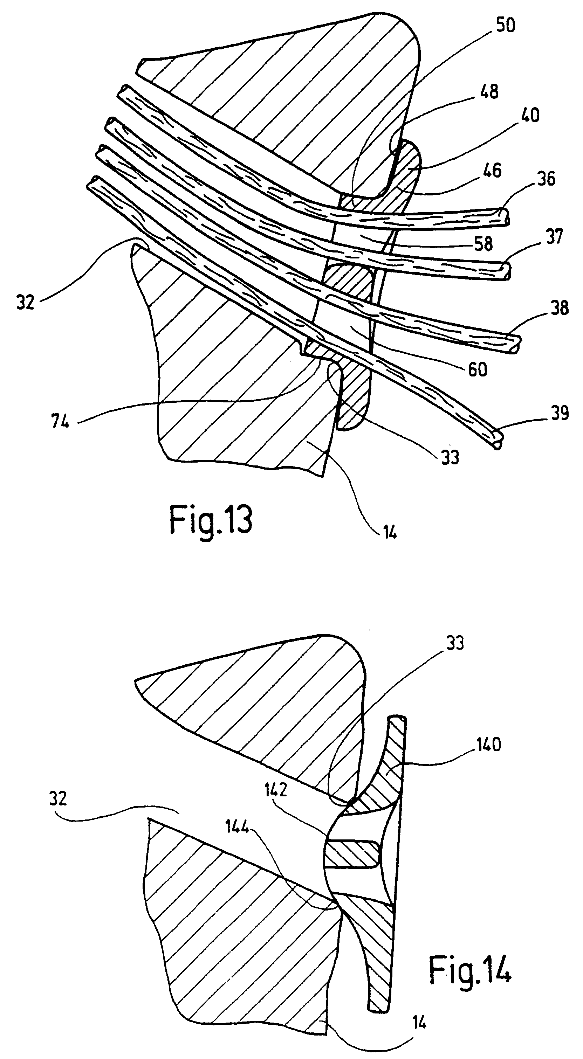

Der vom Körper 46 des Buttons 40 vorspringende Hals 50 ist dazu vorgesehen, um in einer Einsenkung 74 (siehe Fig. 1 und 13) im Bereich der Mündung 33 des Bohrkanales eingesetzt zu werden. Die Länge 72 des Halses 50 entspricht dabei der Tiefe der Einsenkung 74, der lichte Durchmesser der Ensenkung 74 entspricht dem Außendurchmesser des zylindrischen Zapfens 52, so daß dieser passend in die Einsenkung 74 aufgenommen werden kann.

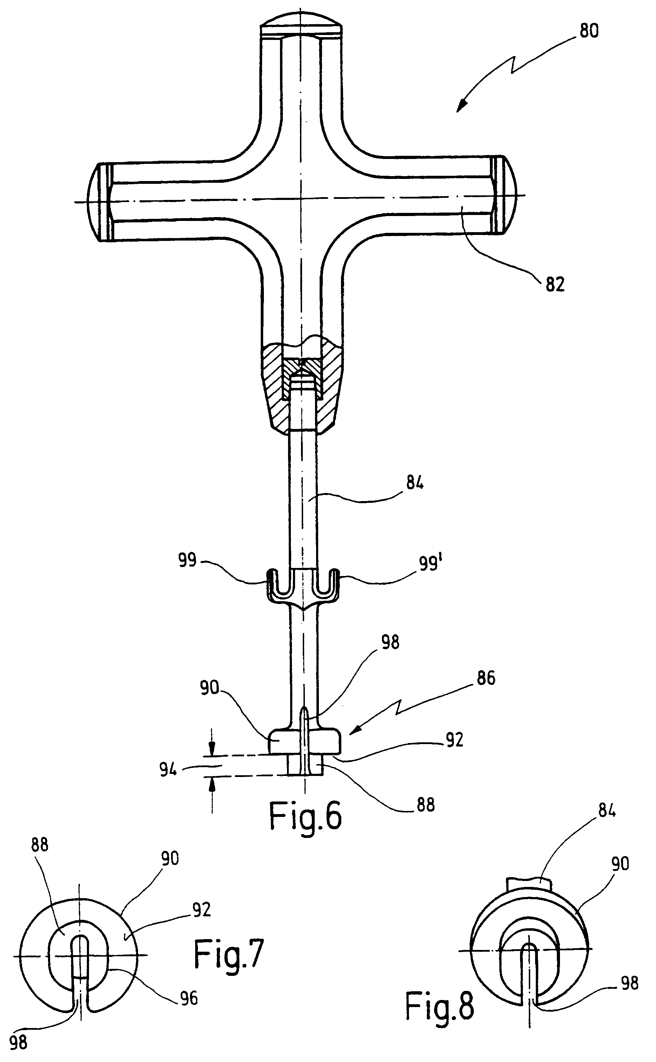

Wie zuvor erwähnt, wurde der Knochenkanal 32 durch die Tibia 14 durch eine Bohrung mit einem Bohrwerkzeug bewerkstelligt. Zur Bewerkstelligung der an die Kontur des Zapfens 52 des Buttons 40 angepaßten Einsenkung 74 ist ein in den Fig. 6 bis 8 dargestelltes Setzgerät 80 vorgesehen.

Das Setzgerät 80 weist dazu einen etwa kreuzartigen Griff 82 auf, von dem distal eine Stange 84 vorspringt, an der am äußeren distalen Ende ein Werkzeug 86 vorhanden ist.

Das Werkzeug 86 weist einen Zapfen 88 auf, dessen proximales Ende von einem Ringflansch 90 umrundet ist, dessen distale Ringfläche als Anschlag 92 dient.

Der Zapfen 88 weist eine Länge 94 auf, die der Länge 72 des zapfenartigen Halses 50 des Buttons 40 entspricht.

Aus der stirnseitigen Ansicht von Fig. 7 bzw. 8 ist zu entnehmen, daß der Zapfen 88 die Kontur eines Ovals 96 aufweist. Seitlich im Bereich des Werkzeuges 86 ist ein sich in Längsrichtung der Stange 84 erstreckender Schlitz 98 vorgesehen. Wie insbesondere aus Fig. 7 zu entnehmen, reicht der Schlitz 98 radial über den Ringflansch 90 bis in den Bereich des Zapfens 88 hinein.

Proximal etwas beabstandet vom Werkzeug 86 sind an der Stange 84 noch zwei Haken 99, 99' vorgesehen.

Die Bewerkstelligung der Einsenkung 74, in die der zapfenartige Hals 50 des Buttons 40 eingeschoben wird, erfolgt mit dem Setzgerät 80 wie folgt. Die vier Fixationsfäden 36, 37, 38 und 39, die aus dem Knochenkanal 32 an der Außenseite der Tibia 14 herausragen, werden gebündelt, das Setzgerät 80 angesetzt, die gebündelten Fixationsfäden seitlich in den Schlitz eingelegt und in die Haken 99 bzw. 99' eingehängt. Das Setzgerät 80 bzw. dessen Werkzeug 36 wird an der ovalen Mündung 33 angesetzt, und das Setzgerät 80 wird, falls notwendig, eingeschlagen, bspw. mittels eines Hammers. Dabei wird so weit eingeschlagen, bis der Anschlag 92 an der Außenseite der Tibia 14 anliegt. Die Ausrichtung der Längsachse der Stange 84 des Setzgerätes 80 ist derart, daß diese etwa in einem rechten Winkel zu der die Mündung 33 des Knochenkanals 32 umgebenden Knochenfläche steht. Nach Einschlagen des Setzgerätes 18 wird dieses gedreht, so daß das Werkzeug 86 durch Drehen die Einsenkung 74 im Knochen ausformt.

Nach Ausformen der Einsenkung 74 wird das Setzgerät 80 abgezogen, und auf die vier Fixationsfäden 36 bis 39 wird ein Button 40 aufgefädelt, wie das in Fig. 13 dargestellt ist. Daraus ist ersichtlich, daß zwei Fixationsfäden 36 und 37 durch die Öffnung 58 hindurchgefädelt wurden, zwei andere Fixationsfäden 38 und 39 durch die Öffnung 60.

Der Button 40 sitzt mit seinem zapfenartigen Hals 50 passend in der Einsenkung 74 im Bereich der Mündung 33 des Knochenkanales 32. Zugleich liegt der Körper 46 des Buttons flächig über seine Unterseite 48 an der Außenseite des Bereiches der Tibia 14 an, die die Mündung 33 umgibt. Aus der Schnittdarstellung von Fig. 13 ist besonders eindrucksvoll zu sehen, daß der Button 40 sowohl im Bereich des Körpers 46 als auch im Bereich des zapfenartigen Halses 50 über einen großen Flächenbereich passend und exakt ausgerichtet und vor allem kippsicher sitzt.

Dies ist gerade ein Problem eines Buttons 140 entsprechend dem eingangs erwähnten Stand der Technik, wie er in Fig. 14 dargestellt ist. Ein derartiger aus dem Stand der Technik bekannter Button 140 weist einen höckerartigen Vorsprung 142 auf, der in die Mündung des Knochenkanales 32 mehr oder weniger weit hinein reicht. Darüber hinaus ist ersichtlich, daß ein solch angelegter Button 140 etwa im Bereich einer Linie 144, die dem äußeren Rand des Knochenkanales 32 entspricht, anliegt. Dadurch ist ein Verkippen und Verkanten des Buttons 140 möglich.

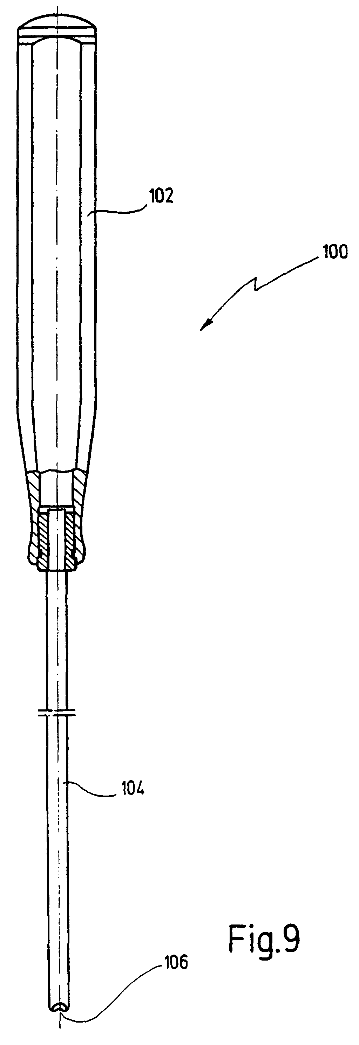

Zurückkehrend zu der Darstellung von Fig. 13, werden nunmehr die Fixationsfäden unter Spannen des Sehnenersatzes 16 miteinander verknotet. Zur Erleichterung des Verknotungsvorganges und zum Halten sowohl des Buttons 40 als auch eines dadurch hindurchgezogenen Fixationsfadens ist der in Fig. 9 vorgesehene Knotenhalter 100 vorgesehen. Dieser weist einen Griff 102 auf, von dem distal ein Stab 104 vorsteht, dessen äußeres distales Ende mit einer Kerbe 106 versehen ist.

Über die Kerbe 106 wird der Knotenhalter an entweder einen Fixationsfaden bzw. einen schon bereits ausgebildeten Knoten angelegt, um diesen Faden oder diesen Knoten mit weiteren Fäden zu verknoten. Ist dieser Vorgang beendet, resultiert ein Gebilde, wie es in Fig. 1 dargestellt ist. Anhand der zusätzlich angebrachten Schlaufe 42 ist es nunmehr möglich, die Spannung des Sehnenersatzes 16 zu überprüfen.

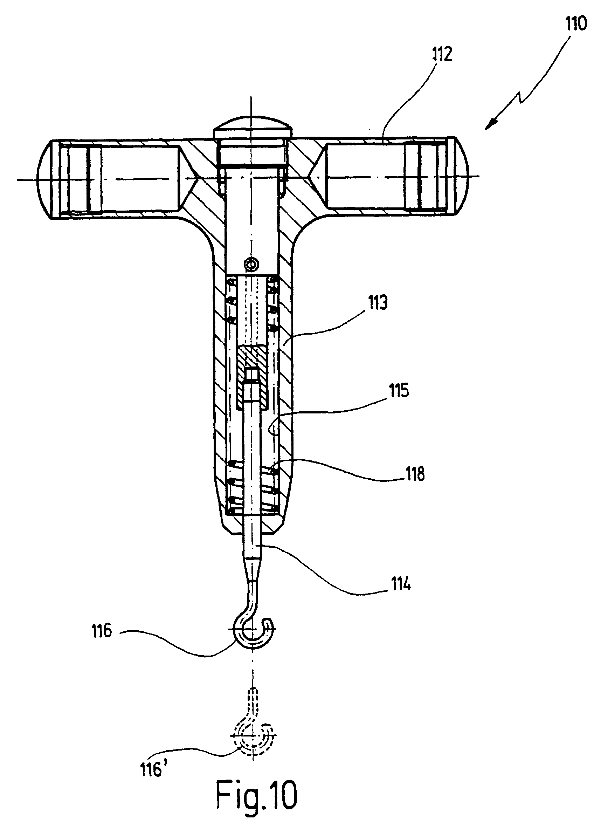

Dazu wird ein in Fig. 10 dargestelltes Tensiometer 110 herangezogen.

Das Tensiometer 110 weist einen Griff 112 auf, von dem distal ein Hohlschaft 113 vorspringt.

Im Hohlschaft 113 ist eine Stange 114 aufgenommen, deren äußeres distales Ende mit einem Haken 116 versehen ist. Die Stange 114 ist gegen die Kraft einer Feder 118 aus dem Schaft 113 herausziehbar. Entsprechend hier nicht dargestellte Markierungen erlauben eine Aussage darüber, welche Kraft notwendig war, um die Stange 114 ein bestimmtes Maß aus dem Schaft 113 herauszuziehen. Zum Prüfen der Spannung des Sehnenersatzes 16 wird das Tensiometer 110 über dessen Haken 116 in die Schlaufe 42 eingehakt und daran gezogen, bis sich der Button 40 gerade beginnt abzuheben. Die entsprechend ausgezogene Position des Hakens 116 ist in Fig. 10 in gestrichelter Position dargestellt.

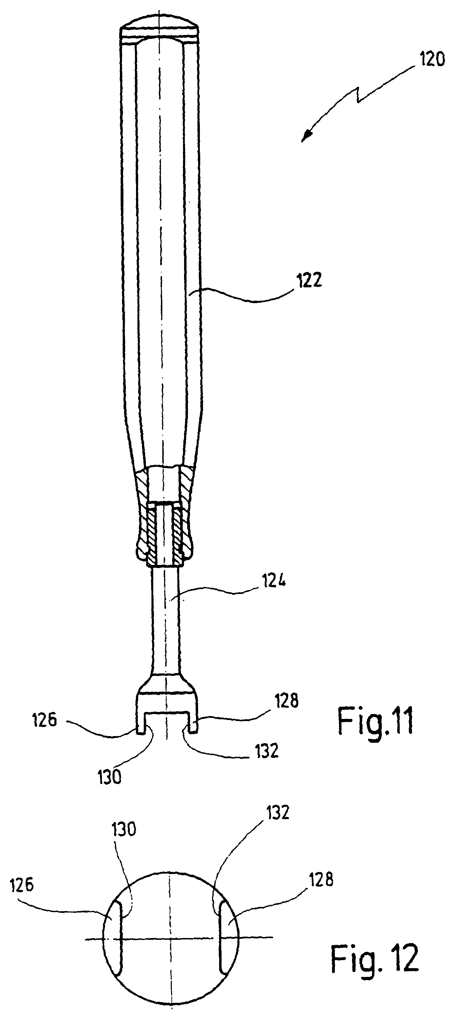

Ist die Spannung des Sehnenersatzes 16 zutreffend, kann der Button 40 in seiner Stellung verbleiben. Ist die Spannung bspw. noch nicht ausreichend, kann der Button 40 verdreht werden, wodurch dann die Fixationsfäden bzw. der Sehnenersatz verdrillt und gespannt wird. Dazu ist der in Fig. 11 und 12 dargestellte Buttondreher 120 vorgesehen.

Der Buttondreher 120 weist einen Griff 122 auf, von dem proximalseitig ein Stab 124 vorsteht. Das distale Ende des Stabes 124 ist mit zwei Backen 126 bzw. 128 versehen, deren innere diametral gegenüberliegende geradlinige Arbeitsflächen 130 bzw. 132 derart ausgebildet bzw. beabstandet sind, daß diese von außen an die entsprechenden Kanten 54 und 56 (siehe insbesondere Fig. 2) des Buttons 40 angesetzt werden können. Durch Verdrehen eines solch angesetzten Buttondrehers 120 wird der Button 40 gedreht und damit der Sehnenersatz 16 gespannt.

Durch den insbesondere aus Fig. 13 ersichtlichen großflächigen Sitz des Buttons 40 ist ein ausreichender Reibschluß gewährleistet, der sicherstellt, daß ein mittels des Buttondrehers 120 verdrehter Button 40 dann an Ort und Stelle verbleibt und sich nicht durch die Rückstellkraft aufgrund des Verdrillens weiter verdreht. Dies zeigt auch den Vorteil bezüglich der Konstruktion der eingangs genannten EP-A-0 865 774, die mit nagelartigen Verankerungen arbeitet und somit ein nachträgliches Drehen und Spannen des Sehnenersatzes nicht mehr möglich macht.

## Patentansprüche

1. Instrumentarium zum Implantieren eines Sehnenersatzes (16), insbesondere eines Kreuzbandersatzes in einem Kniegelenk (10), mit einem Button (40), der an eine äußere Mündung (33) eines Knochenkanales (32) anlegbar ist, in dem der Sehnenersatz (16) angeordnet ist, wobei der Button (40) Öffnungen (58, 60) aufweist, durch die Fixationsfäden (36-39) des Sehnenersatzes (16) hindurchziehbar sind, und wobei der Button (40) einen mittigen Vorsprung aufweist, der bei angelegtem Button (40) in die Mündung (33) des Knochenkanales (32) hineinragt, **dadurch gekennzeichnet, daß** der Vorsprung als zylindrischer Zapfen (52) ausgebildet ist, der in eine diesem angepaßte Einsenkung (74) an der Mündung (33) des Knochenkanales (32) einsetzbar ist, daß der Button (40) einen plattenförmigen Körper (46) aufweist, von dem der zylindrische Zapfen (52) vorspringt, wobei der Button (40) über den plattenförmigen Körper (46) flächig und reibschlüssig im Bereich um die Mündung (33) des Knochenkanales anliegen kann, und daß der plattenförmige Körper (46) Kanten (54, 56) aufweist, an die ein Werkzeug (120) zum Drehen des Buttons (40) ansetzbar ist.

2. Instrumentarium nach Anspruch 1, **dadurch gekennzeichnet, daß** der plattenförmige Körper (46) zwei gegenüberliegende Kanten (54, 56) aufweist.

3. Instrumentarium nach Anspruch 1 oder 2, **dadurch gekennzeichnet, daß** der Button (40) auf der Seite (61), die dem zylindrischen Zapfen (52) gegenüberliegt, im Bereich der Öffnungen (58, 60) eine Mulde (62) aufweist.

4. Instrumentarium nach Anspruch 3, **dadurch gekennzeichnet, daß** die Mulde (62) in sanften Krümmungen (64, 66) in die Öffnungen (58, 60) übergeht.

5. Instrumentarium nach einem der Ansprüche 1 bis 4, **dadurch gekennzeichnet, daß** wenigstens zwei Öffnungen (58, 60) im Button (40) vorhanden sind.

6. Instrumentarium nach einem der Ansprüche 1 bis 5, **dadurch gekennzeichnet, daß** der zylindrische Zapfen (52) von einer flachen Seite (48) des Buttons (40) vorspringt.

7. Instrumentarium nach Anspruch 6, **dadurch gekennzeichnet, daß** der zylindrische Zapfen (52) unter einem solchen Winkel von der flachen Seite (48) vorspringt, daß bei angelegtem Button (40) diese Seite (48) flächig an einer Knochenfläche, die den Knochenkanal (32) umgibt, anliegt und der zylindrische Zapfen (52) dabei passend in der Einsenkung (74) sitzt.

8. Instrumentarium nach einem der Ansprüche 1 bis 7, **dadurch gekennzeichnet, daß** ein Setzgerät (80) vorgesehen ist, das ein distales Werkzeug (86) aufweist, mittels dem die Einsenkung (74) für den zylindrischen Zapfen (52) herstellbar ist.

9. Instrumentarium nach Anspruch 8, **dadurch gekennzeichnet, daß** das Werkzeug (86) distal einen vorstehenden Zapfen (88) aufweist, dessen Länge (94) in etwa der Länge (72) des zylindrischen Zapfens (52) des Buttons (40) entspricht.

10. Instrumentarium nach Anspruch 9, **dadurch gekennzeichnet, daß** der Zapfen (88) an seinem proximalen Ende einen Anschlag (92) aufweist.

11. Instrumentarium nach Anspruch 10, **dadurch gekennzeichnet, daß** der Anschlag (92) als Ringflansch (90) ausgebildet ist.

12. Instrumentarium nach einem der Ansprüche 9 bis 11, **dadurch gekennzeichnet, daß** die Kontur des Zapfens (88) als ein Oval (96) ausgebildet ist.

13. Instrumentarium nach einem der Ansprüche 8 bis 12, **dadurch gekennzeichnet, daß** im Bereich des Werkzeuges (86) zumindest ein seitlicher Längsschlitz (98) vorgesehen ist, in den die Fixationsfäden (36-39) einlegbar sind.

14. Instrumentarium nach einem der Ansprüche 8 bis 13, **dadurch gekennzeichnet, daß** abseits des Werkzeuges (86) Haken (99, 99') zum Einhängen der Fixationsfäden (36-39) vorgesehen sind.

15. Instrumentarium nach einem der Ansprüche 8 bis 14, **dadurch gekennzeichnet, daß** das Setzgerät (80) einen kreuzartigen Griff (82) aufweist.

16. Instrumentarium nach einem der Ansprüche 1 bis 7, **dadurch gekennzeichnet, daß** ein Knotenhalter (100) vorgesehen ist, der einen Griff (102) und einen davon distal vorstehenden Stab (104) aufweist, dessen distales Ende mit einer Kerbe (106) versehen ist, um die Fixationsfäden (36-39) beim Verknoten am Button (40) zu halten.

17. Instrumentarium nach einem der Ansprüche 1 bis 7, **dadurch gekennzeichnet, daß** ein Tensiometer (111) vorgesehen ist, dessen distales, gegen Federkraft ausziehbares Ende (114) mit einem Haken (116) versehen ist, der in eine am Button (40) ausgebildete Schlaufe (42) aus Fixationsfäden (36, 38) einhängbar ist.

18. Instrumentarium nach einem der Ansprüche 1 bis 7, **dadurch gekennzeichnet, daß** als Werkzeug zum Drehen des Buttons (40) ein Buttondreher (120) vorgesehen ist, der an den Button (40) ansetzbar ist und mit dem der Button (40) drehbar ist.

19. Instrumentarium nach Anspruch 18, **dadurch gekennzeichnet, daß** der Buttondreher (120) Arbeitsflächen (130, 132) aufweist, die an die Kanten (54, 56) des Buttons (40) anlegbar sind.

## Claims

1. Instrumentarium for implantation of a substitute tendon (16), especially a substitute crucial ligament in a knee-joint (10), having a button (40) that can be placed against an outer opening (33) of a bone channel (32) in which the substitute tendon (16) is arranged, the button (40) comprises openings (58, 60) through which fixation threads (36 - 39) of the substitute tendon (16) can be threaded, and the button (40) comprising a central projection that extends into the outer opening (33) of the bone channel (32) when the button (40) is applied against the latter, **characterized in that** the projection is configured as a cylindrical pin (52) that can be fitted in a matching countersunk recess (74) at the outer opening (33) of the bone channel (32), **in that** the button (40) comprises a plate-shaped body (46) with the cylindrical pin (52) projecting therefrom, the button (40) can rest via the plate-shaped body (46) two-dimensionally and with frictional engagement in the area around the outer opening (33) of the bone channel (32), and **in that** the plate-shaped body (46) is provided with edges (54, 56) against which a tool (120) can be applied for rotating the button (40).

2. Instrumentarium of claim 1, **characterized in that** the plate-shaped body (46) is provided with two opposite edges (54, 56).

3. Instrumentarium of claim 1 or 2, **characterized in that** the button (40) is provided with a depression (62) in the area of the openings (58, 60) on the side (61) opposite the cylindrical pin (52).

4. Instrumentarium of claim 3, **characterized in that** the depression (62) verges into the openings (58, 60) via smooth curvatures (64, 66).

5. Instrumentarium of anyone of claims 1 through 4, **characterized in that** at least two openings (58, 60) are provided in the button (40).

6. Instrumentarium of anyone of claims 1 through 5, **characterized in that** the cylindrical pin (52) projects from a plane side (48) of the button (40).

7. Instrumentarium of claim 6, **characterized in that** the cylindrical pin (52) projects from the plane side (48) at such an angle, that when the button (40) is seated the respective side (48) comes to rest two-dimensionally against a bone surface, that surrounds the bone channel (32), and the cylindrical pin (52) comes to fit snuggly in the countersunk recess (74).

8. Instrumentarium of anyone of claims 1 through 7, **characterized in that** a setting device (80) is provided, comprising a distal tool (86) by means of which the countersunk recess (74) can be managed in the bone for the cylindrical pin (52).

9. Instrumentarium of claim 8, **characterized in that** the tool (86) is provided, on its distal side, with a projecting pin (88) the length (94) of which corresponds substantially to the length (72) of the cylindrical pin (52) of the button (40).

10. Instrumentarium of claim 9, **characterized in that** the pin (88) comprises a limit stop (92) on its proximal end.

11. Instrumentarium of claim 10, **characterized in that** the stop (92) is configured as an annular flange (90).

12. Instrumentarium of anyone of claims 9 through 11, **characterized in that** the contour of the pin (88) is designed as an oval contour (96).

13. Instrumentarium of anyone of claims 8 through 12, **characterized in that** a lateral longitudinal slot (98) is provided in the area of the tool (86), into which the fixation threads (36 - 39) can be placed.

14. Instrumentarium of anyone of claims 8 through 13, **characterized in that** hooks (99, 99') for mounting the fixation threads (36 - 39) are provided in a position remote from the tool (86).

15. Instrumentarium of anyone of claims 8 through 14, **characterized in that** the setting device (80) comprises a cross-shaped handle (82).

16. Instrumentarium of anyone of claims 1 through 7, **characterized in that** a knot holder (100) is provided, that comprises a handle (102) and a bar (104) projecting distally therefrom, whose distal end is provided with a notch (106) intended to hold the fixation threads (36 - 39) on the button (40) during knotting.

17. Instrumentarium of anyone of claims 1 through 7, **characterized in that** a tensiometer (111) is provided whose distal end (114), that can be pulled out against the action of a spring, carries a hook (116) that can be engaged in a loop (42) of fixation threads (36 - 39) formed on the button (40).

18. Instrumentarium of anyone of claims 1 through 7, **characterized in that** a button-turning tool (120) is provided that can be applied upon the button (40) and by means of which the button (40) can be turned.

19. Instrumentarium of claim 18, **characterized in that** the button-turning tool (120) comprises working surfaces (130, 132) that can be applied against edges (54, 56) of the button (40).

## Revendications

1. Instrumentation pour l'implantation d'un tendon de remplacement (16), en particulier d'un ligament croisé de remplacement, dans une articulation du genou (10), avec un bouton (40) qui peut être appuyé contre une embouchure extérieure (33) d'un canal osseux (32) dans lequel est disposé le tendon de remplacement (16), le bouton (40) présentant des orifices (58, 60) à travers lesquels on peut tirer les fils de fixation (36 à 39) du tendon de remplacement (16), et le bouton (40) présentant une saillie centrale, qui dépasse dans l'embouchure (33) du canal osseux (32) lorsque le bouton (40) est appuyé, **caractérisée en ce que** la saillie est réalisée comme un tenon cylindrique (52) qui peut être inséré dans un renfoncement (74) adapté à celui-ci au niveau de l'embouchure (33) du canal osseux (32), **en ce que** le bouton (40) présente un corps en forme de plaque (46), à partir duquel le tenon cylindrique (52) fait saillie, le bouton (40) pouvant s'appuyer à travers le corps en forme de plaque (46) de façon plane et par friction dans la zone autour de l'embouchure (33) du canal osseux, et **en ce que** le corps en forme de plaque (46) présente des bords (54, 56) auxquels on peut appliquer un outil (120) permettant de tourner le bouton (40).

2. Instrumentation selon la revendication 1, **caractérisée en ce que** le corps en forme de plaque (46) présente deux bords opposés (54, 56).

3. Instrumentation selon la revendication 1 ou 2, **caractérisée en ce que** le bouton (40) présente une cuvette (62) sur le côté (61) opposé au tenon cylindrique (52), au niveau des orifices (58, 60).

4. Instrumentation selon la revendication 3, **caractérisée en ce que** la cuvette (62) passe aux orifices (58, 60) par de légères courbures (64, 66).

5. Instrumentation selon l'une quelconque des revendications 1 à 4, **caractérisée en ce qu'**au moins deux orifices (58, 60) existent dans le bouton (40).

6. Instrumentation selon l'une quelconque des revendications 1 à 5, **caractérisée en ce que** le tenon cylindrique (52) fait saillie à partir d'un côté plat (48) du bouton (40).

7. Instrumentation selon la revendication 6, **caractérisé en ce que** le tenon cylindrique (52) fait saillie à partir du côté plat (48) sous un angle tel que lorsque le bouton (40) est appuyé, ce côté est appuyé de façon plane contre une surface osseuse entourant le canal osseux (32) et le tenon cylindrique (52) est alors logé de façon correspondante dans le renfoncement (74).

8. Instrumentation selon l'une quelconque des revendications 1 à 7, **caractérisée en ce qu'**un dispositif de pose (80) est prévu qui présente un outil distal (86) qui permet de réaliser le renfoncement (74) pour le tenon cylindrique (52).

9. Instrumentation selon la revendication 8, **caractérisée en ce que** l'outil (86) présente de façon distale un tenon (88) dont la longueur (94) correspond approximativement à la longueur (72) du tenon cylindrique (52) du bouton (40).

10. Instrumentation selon la revendication 9, **caractérisée en ce que** le tenon (88)présente à son extrémité proximale une butée (92).

11. Instrumentation selon la revendication 10, **caractérisée en ce que** la butée (92) est réalisée comme une bride annulaire (90).

12. Instrumentation selon l'une quelconque des revendications 9 à 11, **caractérisée en ce que** le contour du tenon (88) est réalisé comme un ovale (96).

13. Instrumentation selon l'une quelconque des revendications 8 à 12, **caractérisée en ce qu'**au niveau de l'outil (86), au moins une fente longitudinale latérale (98) est prévue dans laquelle on peut placer les fils de fixation (36 à 39).

14. Instrumentation selon l'une quelconque des revendications 8 à 13, **caractérisée en ce qu'**à l'écart de l'outil (86), des crochets (99, 99') pour accrocher les fils de fixation (36 à 39) sont prévus.

15. Instrumentation selon l'une quelconque des revendications 8 à 14, **caractérisée en ce que** le dispositif de pose (80) présente une poignée en croix (82).

16. Instrumentation selon l'une quelconque des revendications 1 à 7, **caractérisée en ce qu'**un support de noeud (100) est prévu qui présente une poignée (102) et une tige (104), faisant saillie de façon distale à partir de celle-ci, dont l'extrémité distale est munie d'une encoche (106) pour maintenir les fils de fixation (36 à 39) lorsque le bouton (40) est attaché.

17. Instrumentation selon l'une quelconque des revendications 1 à 7, **caractérisée en ce qu'**un tensiomètre (111) est prévu dont l'extrémité (114) distale, extensible contre une force de ressort, est munie d'un crochet (116) qui peut s'accrocher dans une boucle (42) en fils de fixation (36, 38) réalisée sur le bouton (40).

18. Instrumentation selon l'une quelconque des revendications 1 à 7, **caractérisée en ce qu'**un tournebouton (120) est prévu comme outil pour tourner le bouton (40), le tournebouton pouvant être appliqué au bouton (40) et permettant de tourner le bouton (40).

19. Instrumentation selon la revendication 18, **caractérisée en ce que** le tournebouton (120) présente des surfaces opérationnelles (130, 132) qui peuvent être appuyées contre les bords (54, 56) du bouton (40).
